(19) Europäisches Patentamt
European Patent Office
Office européen des brevets

(11) **EP 3 372 686 B1**

(12) **EUROPEAN PATENT SPECIFICATION**

(45) Date of publication and mention
of the grant of the patent:
**20.05.2020 Bulletin 2020/21**

(51) Int Cl.:
*C12N 15/12* [(2006.01)] *A61K 48/00* [(2006.01)]
*A61P 35/00* [(2006.01)]

(21) Application number: **15907626.4**

(86) International application number:
**PCT/CN2015/093909**

(22) Date of filing: **05.11.2015**

(87) International publication number:
**WO 2017/075784 (11.05.2017 Gazette 2017/19)**

(54) **BIOMARKER FOR DETECTION OF LUNG ADENOCARCINOMA AND USE THEREOF**

BIOMARKER FÜR DEN NACHWEIS VON LUNGENADENOKARZINOM UND VERWENDUNG DAVON

BIOMARQUEUR DE DÉTECTION DU CANCER DU POUMON ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.09.2018 Bulletin 2018/37**

(73) Proprietor: **BGI Shenzhen**
**Shenzhen, Guangdong 518083 (CN)**

(72) Inventors:
• **WU, Kui**
**Shenzhen**
**Guangdong 518083 (CN)**
• **LI, Fuqiang**
**Shenzhen**
**Guangdong 518083 (CN)**
• **HOU, Yong**
**Shenzhen**
**Guangdong 518083 (CN)**
• **ZHU, Shida**
**Shenzhen**
**Guangdong 518083 (CN)**
• **YE, Xiaofei**
**Shenzhen**
**Guangdong 518083 (CN)**
• **LIANG, Yan**
**Shenzhen**
**Guangdong 518083 (CN)**

(74) Representative: **HGF Limited**
**8th Floor**
**140 London Wall**
**London EC2Y 5DN (GB)**

(56) References cited:
WO-A1-2011/035433    WO-A1-2014/151117
WO-A1-2014/172456    WO-A2-2012/031008
WO-A2-2013/152989    CN-A- 104 818 334
TW-A- 201 311 906    US-A1- 2005 112 707
US-A1- 2015 153 346

• KUI WU ET AL: "Frequent alterations in cytoskeleton remodelling genes in primary and metastatic lung adenocarcinomas", NATURE COMMUNICATIONS, vol. 6, no. 1, 1 December 2015 (2015-12-01), XP055486007, DOI: 10.1038/ncomms10131

EP 3 372 686 B1

## Description

## FIELD

The present disclosure relates to the field of biomedicine, particularly to a biomarker for detecting lung adenocarcinoma and use thereof; more particularly to a method for determining *in vitro* whether a subject suffers from lung adenocarcinoma,

## BACKGROUND

With the completion of the Human Genome Project, Next Generation Sequencing provides a novel tool for molecular mechanism research in tumor formation, development and metastasis, which has become an important research strategy in the field of clinical oncology to predict, interfere with, diagnose and treat diseases based on investigation of relation between gene mutation and carcinogenesis by genome patterns in cancer patients. Lung cancer is one of the most malignant tumors that threatens human health and life with fastest increasing morbidity and mortality. In recent years, the morbidity of lung cancer has been rising over time worldwide, especially in China. Lung cancer mainly includes non-small cell lung cancer (NSCLC) (85%) and small cell lung cancer (SCLC), and lung adenocarcinoma is the most common histological type of NSCLC, resulting in over 500,000 deaths annually in the world.

With greater improvement in surgical therapy though, the assistant role of molecular subtyping, targeted therapy and diagnostic tests based on genetic mutation in lung adenocarcinoma in the clinic are still limited by (1) lack of effective tests for early diagnosis, with metastasis already started in most patients once under diagnosis; (2) lack of known effective targetable driver genes for most patients; (3) heterogeneity and complexity of tumors; (4) undetermined mechanism for malignant metastasis.

By focusing on the genomic landscape of lung adenocarcinoma, recent researchers have identified various potential cancer-driver genes, by which targeting therapies have been developed for several activated oncogenes such as EGFR, ERBB2 and BRAF and translocations or fusions involving ALK, ROS1 and RET. However, most of these studies mainly focused on tumor samples obtained from European or North American patients, and the majority of specimens were collected at early disease stages, thus of low specificity for the East Asian population because of distinct ethnic populations. Moreover, there exists a tremendous challenge in diagnosing and treating advanced lung adenocarcinoma where metastasis has already occurred, due to insufficient systematic exploration.

## SUMMARY

Embodiments of the present disclosure aim to solve at least one of the problems existing in the related art, or to provide at least a commercial choice.

Based on discoveries of high morbidity of lung adenocarcinoma among East Asians and potential genetic heterogeneity existing among different ethnic populations, the present inventors have made a comprehensive genetic analysis of East Asians, including genetic profiles for patients with advanced lung adenocarcinoma, especially those diagnosed with metastatic lung adenocarcinoma. With extensive research in genetic mutation using Next Generation Sequencing, the present inventors have identified biomarkers useful in detection of lung adenocarcinoma in early stage, thus enriching the lung adenocarcinoma-driver genes profile and promoting assistant therapy and diagnostic tests for lung adenocarcinoma, e.g., providing biomarkers useful in pathogenesis investigation, prognosis evaluation and metastasis progress, for effective detection of lung adenocarcinoma formation and development; improving understanding of metastasis development; and providing assistant guidance for diagnosis and therapy in the future for metastatic lung adenocarcinoma.

With comprehensive analysis of 335 primary lung adenocarcinoma samples, 35 lymph node metastases samples, and lung adenocarcinoma-free tissue sample, the present inventors integrate mutation rate, gene sequence length, biological function and the like to analyze the mutated genes. These analyses revealed 13 statistically-significant mutated genes in lung adenocarcinoma samples. The mutated genes are TP53, EGFR, LRP1B, KRAS, PTPRD, STK11, SMAD2, PIK3CA, BRAF, FLT1, RHPN2, GLI3 and MRC2, which are also referred to pathogenic genes herein.

The present inventors identified that these genes mutate at a considerable mutation rate for populations with lung adenocarcinoma, especially for the East Asian population, suggesting that these genes are correlated to lung adenocarcinoma pathogenesis, thus having potential in direct or assistant diagnostic tests for lung adenocarcinoma onset. Therefore, these genes can be used as biomarkers for lung adenocarcinoma, i.e., predicting likelihood of lung adenocarcinoma pathogenesis, e.g., assisting in determination of lung adenocarcinoma, or assisting in diagnostic tests for lung adenocarcinoma in early stage.

It has been discovered by the present inventors for the first time that three genes, RHPN2, GLI3 and MRC2, are lung adenocarcinoma-driver genes highly correlated to lung adenocarcinoma, especially closely correlated to lung adenocarcinoma formation and development. Such three lung adenocarcinoma-driver genes are useful in direct or assistant diagnostic test for lung adenocarcinoma, which has not been reported so far.

In a first aspect the present invention provides a method for determining *in vitro* whether a subject suffers from lung adenocarcinoma, comprising: detecting whether the MRC2 gene of lung cells or tissue of the subject contains a mutation; and determining that the subject suffers from lung adenocarcinoma when the MRC2 gene contains said mutation.

In a second aspect of the present invention, there is provided a device for determining whether a subject suffers from lung adenocarcinoma. Said device is suitable to implement the steps of the method described in the first aspect, and the device comprises the following units: a mutation detecting unit, configured to detect whether a MRC2 gene contains a mutation; and a determining unit, connected to the mutation detecting unit and configured to determine that the subject suffers from lung adenocarcinoma when the MRC2 gene contains said mutation.

Also now disclosed is a panel of lung adenocarcinoma-driver genes including the RHPN2, GLI3 and MRC2 genes. Such a panel of lung adenocarcinoma-driver genes may be used in treating lung adenocarcinoma and/or preparing a medicament for treating lung adenocarcinoma.

Thus also now disclosed is a method for determining *in vitro* whether a subject suffers from lung adenocarcinoma, including: detecting whether a panel of lung adenocarcinoma-driver genes including the RHPN2, GLI3 and MRC2 genes each contains a mutation; and determining that the subject suffers from lung adenocarcinoma when any of the panel of lung adenocarcinoma-driver genes contains a mutation, wherein the panel of lung adenocarcinoma-driver genes is from lung cells or tissues of the subject.

Also disclosed is a device for implementing part or all steps of such a method for determining *in vitro* whether a subject suffers from lung adenocarcinoma, the device including the following units: a mutation detecting unit, configured to detect whether a panel of lung adenocarcinoma-driver genes including the RHPN2, GLI3 and MRC2 genes each contains a mutation; and a third determining unit, connected to the mutation detecting unit, and configured to determine that the subject suffers from lung adenocarcinoma when any of the panel of lung adenocarcinoma-driver genes contains a mutation, wherein the panel of lung adenocarcinoma-driver genes is from lung cells or tissues of the subject.

By comparison of lung adenocarcinoma sample data from 335 Chinese patients diagnosed with lung adenocarcinoma, the present inventors identified through Kaplan-Meier survival analysis that mutation in a gene being at least one selected from a group consisting of the TP53, LRP1B, STK11, KEAP1, BRAF, MET and MRC2 genes indicates significantly shorter survival time over a wild-type patient. Said "wild-type patient" herein refers to a patient with lung adenocarcinoma in the absence of mutation in any of the above seven genes. Any one of the above seven genes containing a mutation can be used as a biomarker for assisting prediction of survival in clinical practice.

In a further aspect of the present invention, there is thus provided a method for predicting prognosis of a patient who has undergone treatment for lung adenocarcinoma, comprising: detecting whether a mutation is present in the MRC2 gene of lung cells or tissue of the patient; and determining that the patient is in poor prognosis when the mutation is present in the gene. Said "poor prognosis" means the patient is of shorter survival time than a wild-type patient after treatment for lung adenocarcinoma. Said "wild-type patient" herein refers to a patient with lung adenocarcinoma in the absence of mutation in any of the seven genes: TP53, LRP1B, STK11, KEAP1, BRAF, MET and MRC2.

In a still further aspect of the present invention, there is provided a device for predicting prognosis of a patient who has undergone treatment for lung adenocarcinoma. Said device is suitable to implement the steps of the method for predicting prognosis of a patient who has undergone treatment for lung adenocarcinoma according to the invention as described above and includes the following units: a mutation detecting unit, configured to detect a mutation in the MRC2 gene of lung cells or tissue of the patient; and a determining unit, connected to the mutation detecting unit and configured to determine that the patient is in poor prognosis when the mutation is present in the MRC2 gene.

## DESCRIPTION OF DRAWINGS

The above and/or additional aspects and advantages of the present disclosure will become apparent and easily understood from the description of embodiments in combination with the accompanying drawings, in which:

Figure 1 shows mutation rate in genes related to lung adenocarcinoma from various lung adenocarcinoma patients or different samples.

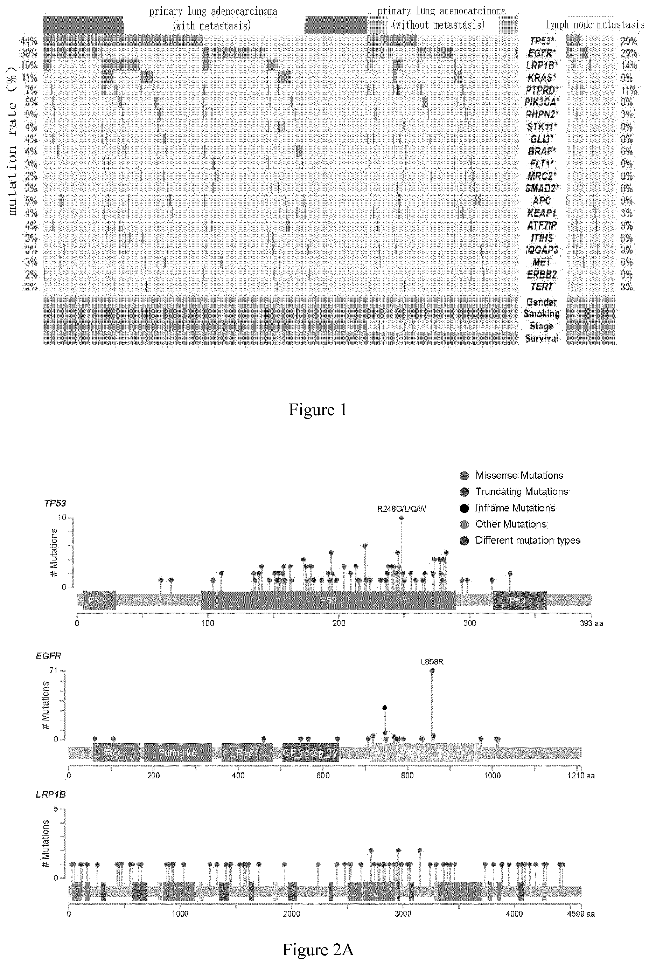

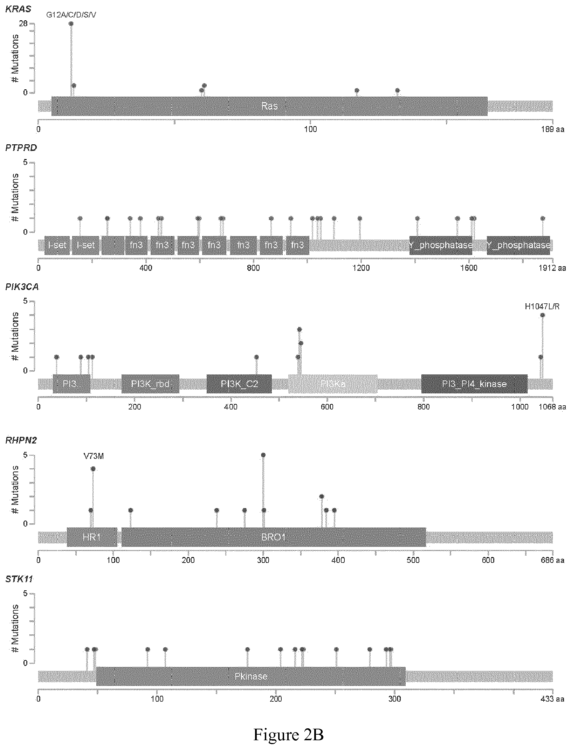

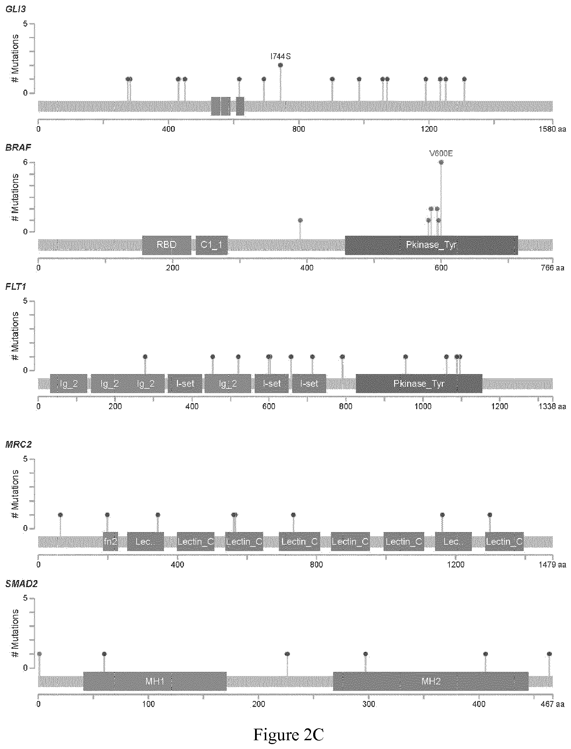

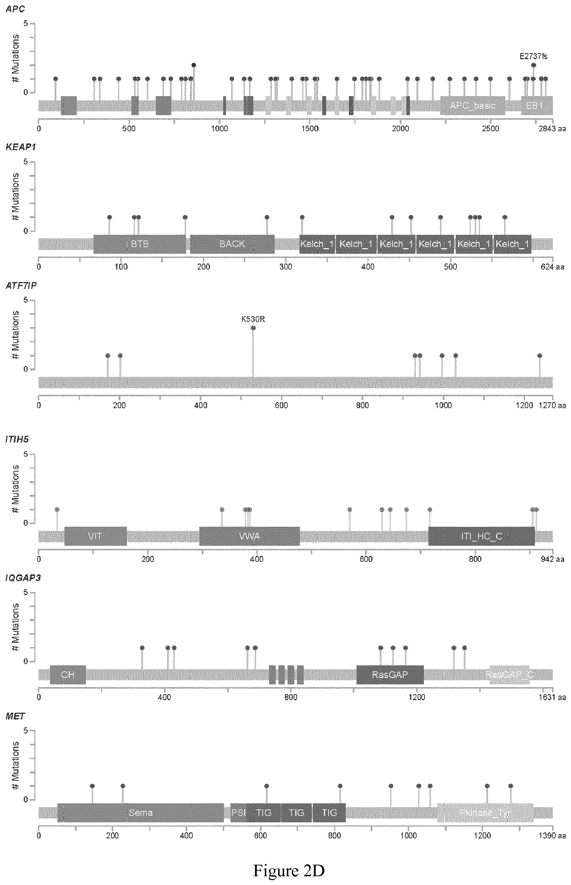

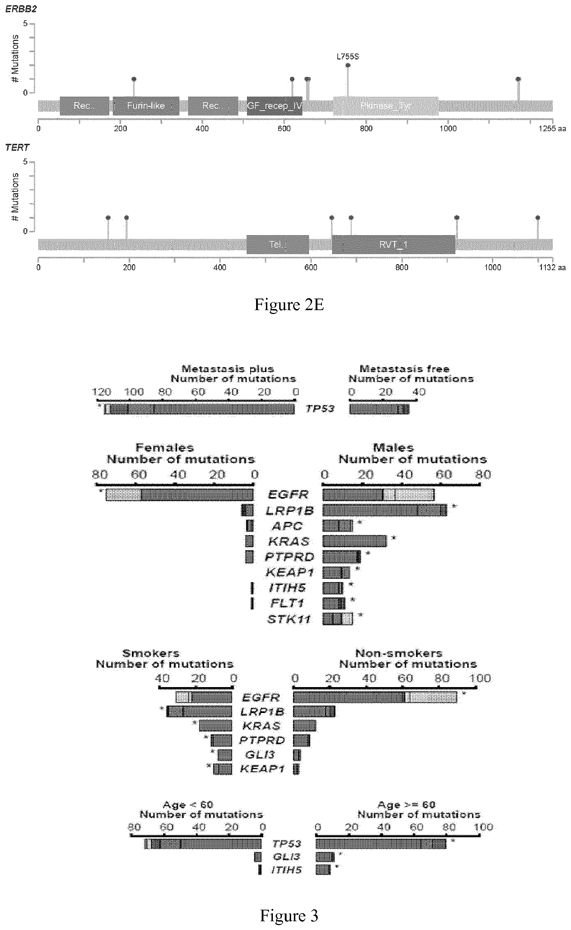

Figure 2 shows mutation types and mutation sites for each gene related to lung adenocarcinoma in which: Figure 2A shows mutation types and mutation sites for each of the TP53, EGFR and LRP1B genes; Figure 2B shows mutation types and mutation sites for each of the KRAS, PTPRD, PIK3CA, RHPN2, and STK11 genes; Figure 2C shows mutation types and mutation sites for each of the BRAF, GLI3, FLT1, MRC2 and SMAD2 genes; Figure 2D shows mutation types and mutation sites for each of the APC, KEAP1, ATF7IP, ITIH5, IQGAP3 and MET genes; and Figure 2E shows mutation types and mutation sites for each of the ERBB2 and TERT genes.

Figure 3 shows relation between mutations in individual genes related to lung adenocarcinoma and clinical features.

Figure 4 shows the impact of mutations individually in 7 genes related to lung adenocarcinoma on fraction of survival and overall survival.

Figure 5 shows a flow chart for determining a biomarker related to lung adenocarcinoma in an embodiment of the present disclosure.

## DETAILED DESCRIPTION

The present disclosure is described in detail with reference to the drawings and specific embodiments.

The same or similar elements and the elements having identical or similar functions are denoted by like reference numerals throughout the descriptions. Reference will be made in detail to embodiments of the present disclosure. The embodiments described herein with reference to drawings are explanatory, illustrative, and used to generally understand the present disclosure. The embodiments shall not be construed to limit the present disclosure.

Terms such as "first" and "second" are used herein for purposes of description and are not intended to indicate or imply relative importance or significance, impliedly indicate the quantity of the technical feature referred to, or indicate the ordinal relation of elements or technical features. Thus, the feature defined with "first" and "second" may comprise one or more of this feature. In the description of the present disclosure, "a plurality of' means two or more than two of the feature, unless specified otherwise.

The terms "sequentially connected", "connected", "linked" and the like herein are to be interpreted broadly, unless specifically defined or limited, for example, such terms may be understood to a fixed connection, a removable connection, or an integrally connection; to a mechanical connection or an electrical connection; or a direct connection, an indirect connection through intermediate media, or internal communication of two elements. It will be appreciated that those skilled in the art can understand the specific meanings of the above terms according to the specific context.

The term "mutation" herein refers to a somatic mutation, meaning the cells except for germ cells are mutated.

In an embodiment of the present invention, there is provided is a method for determining *in vitro* whether a subject suffers from lung adenocarcinoma. Said method comprises: detecting whether the MRC2 gene of lung cells or tissue of the subject contains a mutation; and determining that the subject suffers from lung adenocarcinoma when the MRC2 gene contains the mutation..

Next Generation Sequencing may be employed. Specifically, after extraction from the lung cells or tissues, nucleic acids are subjected to library construction for sequencing on a sequencing platform, with sequencing data obtained. Afterwards, the sequencing data is analyzed by aligning to a reference sequence and identifying a mutation in the target gene based on alignment results. The library can be sequenced on an existing sequencing platform; otherwise the library can be constructed in accordance with a selected sequencing platform. The available sequencing platforms include, but are not limited to Complete Genomics (CG) CGA, Illumina/Solexa, Life Technologies/Ion Torrent and Roche 454. Depending on which one is selected, a library consisting of single-end or paired-end reads is constructed. The alignment can be performed with software, such as the Short Oligonucleotide Analysis Package (SOAP), BWA and the like, which are not limited herein. In the process of alignment, depending on settings of alignment parameters, e.g., h (preferably 1 or 2) is set as the allowable maximal mismatches for each read in the sequencing data, a read containing more than h mismatched bases is considered to be an unmapped read to the reference sequence. The alignment results include whether the read can be mapped to the reference sequence, the position where the read is mapped, the number of the mapped reads at a certain site, the base type at the certain site of the mapped read, and the like. Said reference sequence is a known sequence, which may be any reference sequence of a biological species to which the target individual belongs, such as the published assembled sequence of a genome of the same biological species. If the nucleic acid sample is from a human, its genomic reference sequence (also referred to as the reference genome) can be HG19 provided by the NCBI Database. The mutation type including at least one of SNP, CNV and InDel, may be identified with corresponding known software or program(s) based on the obtained alignment results. For example, an SNP can be identified with software, such as SOAPsnp and GATK according to default parameter settings.

A first reference level can be detected in advance and recorded for future use. In embodiments of the present disclosure, the first reference level is an average mutation level of said biomarker from lung cells or tissues of lung adenocarcinoma-free individuals, which is detected in advance and recorded for future use.

Methods as now disclosed have been developed by the following: based on discovery of high morbidity of lung adenocarcinoma among East Asians and underlying genetic heterogeneity existing among different ethnic backgrounds, the present inventors have made comprehensive analysis of genome patterns of East Asians, including gene profiles for patients with advanced lung adenocarcinoma, especially those diagnosed with metastatic lung adenocarcinoma. With extensive research in genetic mutation using Next Generation Sequencing, the present inventors have identified a biomarker useful in detection of lung adenocarcinoma in early stage, thus enriching the lung adenocarcinoma-driver gene profile and promoting assistant therapy and diagnostic tests for lung adenocarcinoma, e.g., providing a biomarker useful in pathogenesis investigation, prognosis evaluation and metastasis progress, for effective detection of lung adenocarcinoma formation and development; improving understanding of metastasis development; and providing assistant guidance to diagnosis and therapy in the future for metastatic lung adenocarcinoma.

With comprehensive analysis of 335 primary lung adenocarcinoma samples, 35 lymph node metastases samples, and lung adenocarcinoma-free tissue sample, the present inventors integrated mutation rate, gene sequence length, biological function and the like to analysis the mutated genes. These analyses revealed 13 statistically-significant mutated genes in lung adenocarcinoma samples. The mutated genes are the TP53, EGFR, LRP1B, KRAS, PTPRD, STK11, SMAD2, PIK3CA, BRAF, FLT1, RHPN2, GLI3 and MRC2, which are also referred to pathogenic genes herein.

The present inventors discovered that these genes mutate at a considerable mutation rate for populations with lung adenocarcinoma, especially in the East Asian population, referring to Figures 1 and 2, suggesting that these genes are correlated to lung adenocarcinoma pathogenesis, thus having potential in direct or assistant diagnostic tests for lung adenocarcinoma onset. For example, it has been found by the present inventors that a statistically significant mutation is present in the TP53 gene for 44% of the Chinese population with lung adenocarcinoma, suggesting that such a gene can be used as a biomarker for detecting lung adenocarcinoma. It is also demonstrated by the present inventors that a statistically significant mutation is present in the EGFR gene for 39% of the Chinese population with lung adenocarcinoma, which is more common for those who never smoked and females. Besides, as compared with data derived from white people, the mutation in the EGFR gene is at a higher occurring frequency than the KRAS gene. In addition, the mutation in the EGFR gene is shown to mainly occur at the Leu858Arg site and the exon19del site, which are the therapeutically sensitive sites for a tyrosine-kinase inhibitor, suggesting that such a gene can be used as a biomarker in detection or target therapy for lung adenocarcinoma. It is further revealed by the present inventors that a statistically significant mutation is present in the LRP1B gene for 19% of the Chinese population with lung adenocarcinoma, suggesting that such a gene can be used as a biomarker for detecting lung adenocarcinoma. It is further shown by the present inventors that a statistically significant mutation is present in the KRAS gene for 11% of the Chinese population with lung adenocarcinoma, suggesting that such a gene can be used as a biomarker for detecting lung adenocarcinoma. It has further been discovered by the present inventors that a statistically significant mutation is present in the PTPRD gene for 7% of the Chinese population with lung adenocarcinoma, suggesting that such a gene can be used as a biomarker for detecting lung adenocarcinoma. It is further shown by the present inventors that a statistically significant mutation is present in the STK11 gene for 4% of the Chinese population with lung adenocarcinoma, suggesting that such a gene can be used as a biomarker for detecting lung adenocarcinoma. It is further shown by the present inventors that a statistically significant mutation is present in the PIK3CA gene for 5% of the Chinese population with lung adenocarcinoma, suggesting that such gene can be used as a biomarker for detecting lung adenocarcinoma. It is further proved by the present inventors that a statistically significant mutation is present in the RHPN2 gene for 5% of the Chinese population with lung adenocarcinoma, suggesting that such a gene can be used as a biomarker for detecting lung adenocarcinoma. It has further been found by the present inventors that a statistically significant mutation is present in the SMAD2 gene for 2% of the Chinese population with lung adenocarcinoma, suggesting that such a gene can be used as a biomarker for detecting lung adenocarcinoma. It is further demonstrated by the present inventors that a statistically significant mutation is present in the BRAF gene for 4% of the Chinese population with lung adenocarcinoma, suggesting that such a gene can be used as a biomarker for detecting lung adenocarcinoma. It is further revealed by the present inventors that a statistically significant mutation is present in the GLI3 gene for 4% of the Chinese population with lung adenocarcinoma, suggesting that such a gene can be used as a biomarker for detecting lung adenocarcinoma. It is further shown by the present inventors that a statistically significant mutation is present in the FLT1 gene for 3% of the Chinese population with lung adenocarcinoma, suggesting that such a gene can be used as a biomarker for detecting lung adenocarcinoma. It has further been discovered by the present inventors that a statistically significant mutation is present in the MRC2 gene (also referred to uPARAP, Endo 180, or CD280) for 2% of the Chinese population with lung adenocarcinoma, suggesting that such a gene can be used as a biomarker for detecting lung adenocarcinoma. Therefore, each of these genes or combination thereof can be used as biomarker(s) for lung adenocarcinoma, i.e., predicting likelihood of lung adenocarcinoma pathogenesis, e.g., assisting in determination of lung adenocarcinoma, or assisting in diagnostic tests for lung adenocarcinoma in early stage.

Any one, two, three, four, five, six, seven, eight, nine, ten, eleven, twelve and all thirteen genes selected from the group consisting of the TP53, EGFR, LRP1B, KRAS, PTPRD, STK11, SMAD2, PIK3CA, BRAF, FLT1, RHPN2, GLI3 and MRC2 genes may be employed as a biomarker or biomarkers for detecting lung adenocarcinoma.

In an embodiment of the present invention, there is provided a device for determining whether a subject suffers from lung adenocarcinoma. Relying on detecting whether the MRC2 gene of lung cells or tissue of the subject contains a mutation in accordance with the invention as hereinbefore described. Such a device includes the following units: a first detecting unit, configured to detect whether a MRC2 gene contains a mutation; and a determining unit, connected to the mutation detecting unit and configured to determine that the subject suffers from lung adenocarcinoma when the MRC2 gene contains said mutation.

The technical features or advantages of a method of the invention for determining *in vitro* whether a subject suffers from lung adenocarcinoma described are also applicable to the device for such determination. It will be appreciated by those skilled in the art that steps of the method can be implemented by means of sub-units connected to each other. For example, said mutation detecting unit includes a sequencing sub-unit to obtain the sequencing data, and an aligning sub-unit to align the sequencing data to a reference sequence to obtain alignment results.

As hereinbefore indicated, now disclosed is a panel of lung adenocarcinoma-driver genes, including the RHPN2, GLI3 and MRC2. It has been discovered by the present inventors for the first time that such three genes, RHPN2, GLI3 and MRC2, are lung adenocarcinoma-driver genes highly correlated to lung adenocarcinoma, especially closely correlated to lung adenocarcinoma formation and development. Such three lung adenocarcinoma-driver genes are useful in direct or assistant diagnostic tests for lung adenocarcinoma, which have not been reported so far.

The same lung adenocarcinoma-driver genes may find use in treating lung adenocarcinoma and/or in preparation of a medicament for treating lung adenocarcinoma. In this connection, it is of especial note that it has been discovered by the present inventors for the first time that the three genes, RHPN2, GLI3 and MRC2, are lung adenocarcinoma-driver genes closely correlated to lung adenocarcinoma formation and development.

Thus also now disclosed is a method for determining *in vitro* whether a subject suffers from lung adenocarcinoma, including: detecting whether a panel of lung adenocarcinoma-driver genes including the RHPN2, GLI3 and MRC2 genes each contains a mutation; and determining that the subject suffers from lung adenocarcinoma when any of the panel of lung adenocarcinoma-driver genes each contains a mutation, wherein the panel of lung adenocarcinoma-driver genes is from lung cells or tissue of the subject.

In an example of such a method, detecting whether a panel of lung adenocarcinoma-driver genes each contains a mutation further includes detecting whether the RHPN2 gene contains a V73M mutation, where the panel of lung adenocarcinoma-driver genes is from lung cells or tissues of a subject. It has been discovered by the present inventors that the V73M mutation in the RHPN2 gene is only found at a high occurring frequency in populations with lung adenocarcinoma. It should be noted that the V73M mutation in the RHPN2 gene which has never been reported so far can be used as a potential biomarker for mutation related to the lung adenocarcinoma of note, especially as a novel biomarker in diagnosis and therapy of lung adenocarcinoma for the East Asian population.

In another embodiment of the present disclosure, provided is a device for determining whether a subject suffers from lung adenocarcinoma relying on a panel of lung adenocarcinoma-driver genes as hereinbefore described. Such a device includes the following units: a mutation detecting unit, configured to detect whether a panel of lung adenocarcinoma-driver genes including the RHPN2, GLI3 and MRC2 genes each contains a mutation; and a determining unit, connected to the mutation detecting unit, and configured to determine that the subject suffers from lung adenocarcinoma when any of the panel of lung adenocarcinoma-driver genes contains mutation, wherein the panel of lung adenocarcinoma-driver genes is from lung cells or tissue of the subject.

As hereinbefore indicated such detection may further include detecting whether the RHPN2 gene contains a V73M mutation, where the panel of lung adenocarcinoma-driver genes is from lung cells or tissues of a subject.

In another embodiment of the present invention, there is provided a method for predicting prognosis of a patient who has undergone treatment for lung adenocarcinoma, comprising: detecting whether a mutation is present in the MRC2 gene from lung cells or tissue of the patient; and determining that the patient is in poor prognosis when the mutation is present in the gene. Said "poor prognosis" means the patient is of shorter survival than a wild-type patient after treatment for lung adenocarcinoma. Said "wild-type patient" herein refers to a patient with lung adenocarcinoma in the absence of mutation in any of the seven genes: TP53, LRP1B, STK11, KEAP1, BRAF, MET and MRC2.

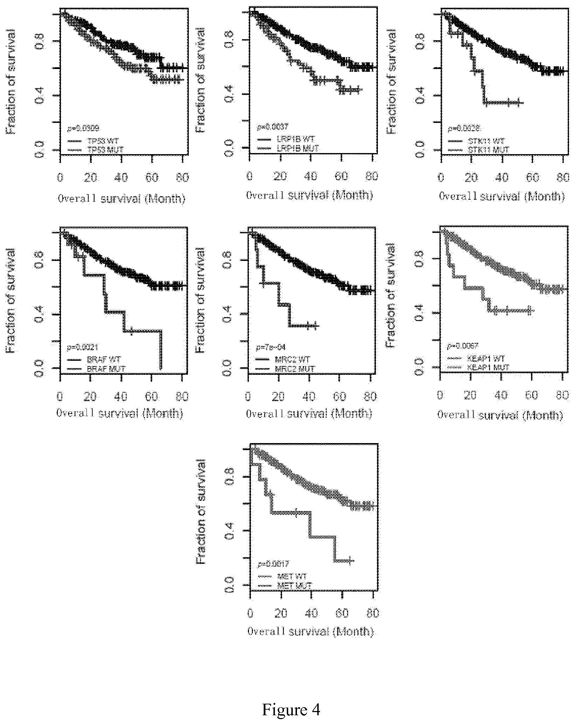

By comparison of lung adenocarcinoma sample data from 335 Chinese patients diagnosed with lung adenocarcinoma, the present inventors identified through Kaplan-Meier survival analysis that mutation in a gene being at least one selected from a group consisting of the TP53, LRP1B, STK11, KEAP1, BRAF, MET and MRC2 genes indicates significantly shorter survival time over a wild-type patient, referring to Figure 4. Said "wild-type patient" herein refers to a patient with lung adenocarcinoma in the absence of mutation in any of the above seven genes. Any one of the above seven genes containing a mutation can be used as a biomarker for assisting prediction of survival in clinical practice.

In this embodiment, mutation can be identified by Next Generation Sequencing. Specifically, after extraction from the lung cells or tissues of one or more patients who have undergone treatment for lung adenocarcinoma, nucleic acids are subjected to library construction for sequencing on a sequencing platform, with sequencing data obtained. Afterwards, the sequencing data is analyzed by aligning to a reference sequence and identifying a mutation level in a target gene based on alignment results. The library can be sequenced on an existing sequencing platform; otherwise the library can be constructed in accordance with a selected sequencing platform. The available sequencing platforms include, but are not limited to Complete Genomics (CG) CGA, Illumina/Solexa, Life Technologies/Ion Torrent and Roche 454, depending on which one is selected, a library consisting of single-end or paired-end reads is constructed. The alignment can be performed with software, such as the Short Oligonucleotide Analysis Package (SOAP), BWA and the like, which are not limited herein. In the process of alignment, depending on settings of alignment parameters, e.g., h (preferably 1 or 2) is set as the allowable maximal mismatches for each read in the sequencing data, a read containing more than h mismatched bases is considered to be an unmapped read to the reference sequence. The alignment results include whether the read can be mapped to the reference sequence, the position where the read is mapped, the number of the mapped reads at a certain site, the base type at the certain site of the mapped read, and the like. Said reference sequence is a known sequence, which may be any reference sequence of a biological species to which the target individual belongs, such as the published assembled sequence of a genome of the same biological species. If the nucleic acid sample is from a human, its genomic reference sequence (also referred to as the reference genome) can be HG19 provided by the NCBI Database. The mutation type including at least one of SNP, CNV and InDel, may be identified with corresponding known software or program(s) based on the obtained alignment results. For example, an SNP can be identified with software, such as SOAPsnp and GATK according to default parameter settings.

In some embodiments of the present disclosure, when referring to determining that the patient is in poor prognosis when the mutation is present in the gene, the mutation is a missense mutation.

In another embodiment of the present invention, there is provided a device suitable for implementing the steps of a method for predicting prognosis of a patient who has undergone treatment for lung adenocarcinoma including the following units: a mutation detecting unit, configured to detect a mutation in the MRC2 gene from lung cells or tissues of the patient; and a determining unit, connected to the mutation detecting unit and configured to determine that the patient is in poor prognosis when the mutation is present in the gene. Said "poor prognosis" means the patient is of shorter survival than the wild-type patient after treatment for lung adenocarcinoma. Said "wild-type patient" herein refers to a patient with lung adenocarcinoma in the absence of mutation in any of the seven genes; TP3, LRP1B, STK11, KEAP1, BRAF, MET and MRC2. It will be appreciated by the skilled in the art that steps of the method for predicting prognosis of a patient who has undergone treatment for lung adenocarcinoma in the above embodiment can be implemented with corresponding functional units each including connected sub-units.

The following examples described herein are explanatory, illustrative, and used to generally understand the present disclosure. The examples shall not be construed to limit the present disclosure.

Unless otherwise specified, the reagents, instruments or software involved in the following examples are all commercially available, for example, a kit for preparing a sequencing library is purchased from the Illumina Corporation, and a library is constructed according to the kit instruction.

**Example**

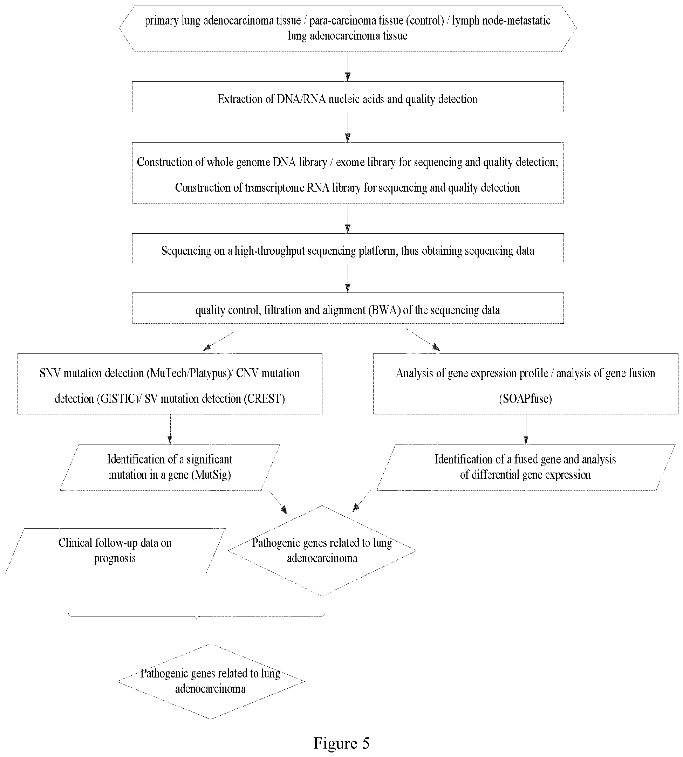

Figure 5 summarizes the flow chart by which the present inventors identified various biomarkers related to lung adenocarcinoma by means of modern molecular biology techniques. In particular, after extraction from samples derived from Chinese populations with lung adenocarcinoma in early stage or with metastatic lung adenocarcinoma, nucleic acids (DNAs or RNAs) were used for nucleic acid library-construction, including fragmentation, end-repairing, adaptor ligation, whole genome amplification and exon capture with a transcriptome library constructed after further amplification. Afterwards, sequencing data, obtained with a high throughput sequencing platform, was processed with biological information software, including alignment of whole genome sequencing data, transcriptome sequencing data, exome sequencing data and the like for the patients diagnosed with lung adenocarcinoma in early stage or with metastatic lung adenocarcinoma, from which it has been demonstrated that those genes highly related to lung adenocarcinoma onset, development and prognosis can be used as effective biomarkers targeting lung adenocarcinoma for Chinese populations.

1. Sample collection and nucleic acids extraction

With patient's informed consent, clinical samples were collected from hospital. In particular, lung adenocarcinoma samples, lymph node samples affected with metastatic lung adenocarcinoma if metastasis occurred, and lung adenocarcinoma-free sample adjacent to the lung adenocarcinoma (para-carcinoma tissue) were collected for pathological analysis to determine the presence of lung adenocarcinoma (primary lung adenocarcinoma or lymph node-metastatic lung adenocarcinoma), e.g., pathological classification; staging as Tumor, Lymph Node and Metastasis (TNM); and tumor cell structures. After collection, the clinical samples were stored in liquid nitrogen at once for future scientific use.

Whole genome DNAs were extracted from the clinical samples with the QIAamp DNA Mini Kit (Qiagen Co Ltd). Total RNAs were extracted from the clinical samples with the TRIzol® reagent (Life Technologies Co Ltd). The obtained DNAs/RNAs samples were determined and qualified by agarose gel electrophoresis and Agilent 2100. In order to guarantee the DNAs/RNAs samples deriving from the same patient, 21 pairs of single nucleotide polymorphism (SNP) sites were validated by Microsatellite Mass Spectrometry.

2. Construction of whole genome, transcriptome and exome libraries and sequencing the same

2.1 Whole genome library

2-3 μg whole genome DNAs sample was randomly fragmented into about 500 bp DNA fragments by a Covarias ultrasonicator, with which a library was constructed in accordance to the standard Illumina Paired-End protocol, including end-repairing in the presence of T4 DNA Polymerase, Klenow fragment, T4 Polynucleotide kinase and nucleotide phosphate; phosphorylation at the 3' terminal of the repaired DNA fragment with the Klenow enzyme (3'-5'exo); and ligation by the T4 DNA ligase to a PE index adaptor that had been synthesized with 5'-methylcytosine in place of cytosine. Purification was performed with the QIAquick PCR Purification Kit (Qiagen Co Ltd) after each of the above steps.

2.2 Whole exome library

3 μg whole genome DNAs sample was firstly fragmented as described in 2.1. The PE index adaptor was ligated to resulting DNA fragments, before those fragments containing exon regions were captured and enriched by the SureSelect Human Whole exon 50Mb Kit under the SureSelect Target Enrichment System, with the whole exome library consisting of 200 bp paired-end reads constructed for Illumina sequencing.

2.3 Transcriptome library

After removal of residual DNA with RNase-free DNase I (New England BioLabs) at 37°C for 30 minutes, 20 μg RNA sample was reverse transcribed into a first strand of cDNA in the presence of oligo(dT) (for capturing mRNA with poly (A) tail), random primers and transcriptases, followed by synthesis of a second strand of cDNA with addition of RNase H (Invitrogen) and DNA polymerase I (New England BioLabs). cDNA library construction was completed according to the manufacturer's instruction.

2.4 Sequencing on a sequencing platform

After construction, the above three sequencing libraries were sequenced separately on the Hiseq2000 sequencing platform at such a high depth that coverage is 50× for the primary lung adenocarcinoma sample and 30× for the para-carcinoma tissue.

3. Data analysis

3.1 Data filtration

After completion of sequencing as described above, raw sequencing data was firstly filtered by the Cutadapt Software (v1.8) (http: //cutadapt.readthedocs.org/en/latest/index.html), including removing sequencing reads containing adaptor sequences and low-quality reads, such as reads containing over 10% unknown bases and reads containing over 50% bases with q value >5. All of the data analysis below is based on data after filtered.

3.2 Data analysis for the whole genome and the whole exome

3.2.1 The PE reads in high quality were aligned with the hg19 reference sequence from the UCSC database (ftp: //hgdownload.cse.ucsc.edu/golden-Path/hg19/bigZips/chromFa.tar.gz) using the BWA software (v0.5.9) (http: //bio-bwa.sourceforge.net/), thereby obtaining an alignment result in a SAM format (http: //samtools.github.io/hts-specs/SAMv1.pdf).

3.2.2 The alignment result in the SAM format was transformed into a BAM format using the Samtools software (v0.1.18) (http: //samtools.sourceforge.net/), followed by ranking according to a coordinate system.

3.2.3 The repeated sequences obtained during PCR amplification were removed using the Picard software (v1.54) (http: //picard.sourceforge.net/).

3.2.4 The sequences adjacent to Indel were locally rearranged using the Genome Analysis Toolkit software (GATK) (v1.0.6076) (https: //www.broadinstitute.org/gatk/).

After the above basic analysis, (1) point mutations were determined using the MutTect software (v1.1.4) (http ://www.broadinstitute.org/cancer/cga/mutect); (2) the point mutations were then analyzed pair-wise between the early-stage lung adenocarcinoma tissue and lung adenocarcinoma-free tissue, as well as between the metastatic lung adenocarcinoma tissue and the adenocarcinoma-free tissue using the Platypus software (v0.7.9.1) (http ://www.well.ox.ac.uk/platypus), with insert and deletion (Indel) determined in the somatic cells; (3) the point mutations were then analyzed pair-wise between the early-ly-stage lung adenocarcinoma tissue and lung adenocarcinoma-free tissue, as well as between the metastatic lung adenocarcinoma tissue and the adenocarcinoma-free tissue using the SegSeq algorithm (http ://www.broadinstitute.org/cancer/cga/segseq), with copy number variation determined in the somatic cells; and (4) the point mutations were then analyzed pair-wise between the early-stage lung adenocarcinoma tissue and lung adenocarcinoma-free tissue, as well as between the metastatic lung adenocarcinoma tissue and the adenocarcinoma-free tissue using the CREST software (v1.0) (www.stjuderesearch.org/site/lab/zhang), with structural variation determined in the somatic cells. All of the above variation determination was annotated using the ANNOVAR software (2011Oct02) (http://annovar.openbioinformatics.org/en/latest/).

Identification of a gene containing a significant mutation related to lung adenocarcinoma was made by analysis with a statistical program, construction of a model for predicting a lung adenocarcinoma-driver gene, calculation of a statistical significance of each mutation and evaluation of correlation between the mutation and the lung adenocarcinoma. Taking together the background distribution of mutation score and test statistics of observed mutation scores across samples, false discovery rate (FDR) was determined using Benjamini-Hochberg method. Only genes with FDR(q-value) < 0.1 were selected for following analysis. A subsequent prediction of significantly mutated genes was performed using Mut-SigCV software(v1.4) (http://www.broadinstitute.org/cancer/cga/mutsig).

3.3 Data analysis of transcriptome

Date alignment

The data after filtration was aligned to the hg19 reference sequence (downloaded from the UCSC database, ftp://hgdownload.cse.ucsc.edu/golden-Path/hg19/bigZips/chromFa.tar.gz) and its transcriptome sequence (downloaded from the UCSC database, ftp://hgdownload.cse.ucsc.edu/goldenPath/hg19/database/refGene.txt.gz) using the SOAP2 software (v2.21) (http: //soap.genomics.org.cn/soapaligner.html), with maximal 5 mismatches allowable for each read. The expression level of each gene was calculated by the RPKM method according to the following formula:

$$RPKM = \frac{10^6 C}{NL/10^3},$$ where C represents the number of reads mapped to a target gene, N represents the total number of reads mapped to a genome, and L represents exon length of the target gene.

4. Results

With comprehensive analysis of 335 primary lung adenocarcinoma samples, 35 lymph node samples affected with metastatic lung adenocarcinoma, and lung adenocarcinoma-free tissue sample, the present inventors integrated occurring frequency, length, biological function and the like of the mutated gene, thus identifying the following biomarkers related to lung adenocarcinoma. Said correlation between these biomarkers and the lung adenocarcinoma was further confirmed with 90% consistent results between detection by the present method and clinical diagnosis. As a result, these biomarkers can be useful in determination on whether lung adenocarcinoma is present and/or whether lung adenocarcinoma has metastasized.

4.1 Pathogenic lung adenocarcinoma-driver genes

Through the above experiments and data analysis with reference to Figures 1 and 2A-C, the present inventors reveal that among all lung adenocarcinoma samples, a statistically-significant mutation is present in any of 13 genes including TP53, EGFR, LRP1B, KRAS, PTPRD, STK11, SMAD2, PIK3CA, BRAF, FLT1, RHPN2, GLI3 and MRC2, which are also referred to pathogenic genes herein. The mutation type of each pathogenic gene is showed in Figure 2.

The present inventors discovered that mutation is present in any these genes at a considerable occurring frequency for the Chinese population with lung adenocarcinoma, suggesting that these genes are correlated to lung adenocarcinoma pathogenesis, thus having potential in diagnostic tests for lung adenocarcinoma onset. Any one or any combination of these 13 genes is useful to detect likelihood of lung adenocarcinoma pathogenesis, e.g., diagnostic tests for lung adenocarcinoma in early stage.

It has been found by the present inventors that a statistically significant mutation is present in the TP53 gene for 44% of the Chinese population with lung adenocarcinoma, referring to Figures 1 and 2A, suggesting that such a gene can be used as a biomarker for detecting lung adenocarcinoma.

It is also demonstrated by the present inventors that a statistically significant mutation is present in the EGFR gene for 39% of the Chinese population with lung adenocarcinoma, referring to Figures 1 and 2A, which is more common for those who never smoked and females. Besides, as compared with data derived from white people, the mutation in the EGFR gene is at a higher occurring frequency than the KRAS gene. In addition, the mutation in the EGFR gene is shown to mainly occur at the Leu858Arg site and the exon19del site, which are the therapeutically sensitive sites for a tyrosine-kinase inhibitor, suggesting that such a gene can be used as a biomarker in detection or target therapy for lung adenocarcinoma.

It is further revealed by the present inventors that a statistically significant mutation is present in the LRP1B gene for 19% of the Chinese population with lung adenocarcinoma, referring to Figures 1 and 2A, suggesting that such a gene can be used as a biomarker for detecting lung adenocarcinoma.

It is further shown by the present inventors that a statistically significant mutation is present in the KRAS gene for 11% of the Chinese population with lung adenocarcinoma, referring to Figures 1 and 2B, suggesting that such a gene can be used as a biomarker for detecting lung adenocarcinoma.

It has further been discovered by the present inventors that a statistically significant mutation is present in the PTPRD gene for 7% of the Chinese population with lung adenocarcinoma, referring to Figures 1 and 2B, suggesting that such a gene can be used as a biomarker for detecting lung adenocarcinoma.

It is further shown by the present inventors that a statistically significant mutation is present in the PIK3CA gene for 5% of the Chinese population with lung adenocarcinoma, referring to Figures 1 and 2B, suggesting that such gene can be used as a biomarker for detecting lung adenocarcinoma.

It is further proved by the present inventors that a statistically significant mutation is present in the RHPN2 gene for 5% of the Chinese population with lung adenocarcinoma, referring to Figures 1 and 2B, suggesting that such a gene can be used as a biomarker for detecting lung adenocarcinoma.

It is further shown by the present inventors that a statistically significant mutation is present in the STK11 gene for 4% of the Chinese population with lung adenocarcinoma, referring to Figures 1 and 2B, suggesting that such a gene can be used as a biomarker for detecting lung adenocarcinoma.

It is further demonstrated by the present inventors that a statistically significant mutation is present in the BRAF gene for 4% of the Chinese population with lung adenocarcinoma, referring to Figures 1 and 2C, suggesting that such a gene can be used as a biomarker for detecting lung adenocarcinoma.

It is further revealed by the present inventors that a statistically significant mutation is present in the GLI3 gene for 4% of the Chinese population with lung adenocarcinoma, referring to Figures 1 and 2C, suggesting that such a gene can be used as a biomarker for detecting lung adenocarcinoma.

It is further shown by the present inventors that a statistically significant mutation is present in the FLT1 gene for 3% of the Chinese population with lung adenocarcinoma, referring to Figures 1 and 2C, suggesting that such a gene can be used as a biomarker for detecting lung adenocarcinoma.

It has further been discovered by the present inventors that a statistically significant mutation is present in the MRC2 gene (also referred to uPARAP, Endo180, or CD280) for 2% of the Chinese population with lung adenocarcinoma, referring to Figures 1 and 2C, suggesting that such a gene can be used as a biomarker for detecting lung adenocarcinoma.

It has been found by the present inventors that a statistically significant mutation is present in the SMAD2 gene for 2% of the Chinese population with lung adenocarcinoma, referring to Figures 1 and 2C, suggesting that such a gene can be used as a biomarker for detecting lung adenocarcinoma.

In addition to the above genes with significant mutation related to lung adenocarcinoma, the present inventors further discovered that other genes containing a non-significant mutation also may be an oncogene in function, including APC, KEAP1, ATF7IP, ITIH5, IQGAP3, MET, ERBB2 and TERT, referring to Figures 1, 2D and 2E.

4.2. Novel lung adenocarcinoma-driver genes

It has been discovered by the present inventors for the first time that the RHPN2, GLI3 and MRC2 genes are three lung adenocarcinoma-driver genes highly correlated to lung adenocarcinoma. Particularly, the V73M mutation in the RHPN2 gene which has never been reported so far can be used as a potential biomarker for mutation related to lung adenocarcinoma of note, especially as a novel biomarker in diagnosis and therapy of lung adenocarcinoma for East Asians.

4.3. Correlation between the mutation in the gene and clinical characteristic

It has been found by the present inventors through Fisher's exact tests that a mutation in the EGFR gene is significantly correlated to non-smoking patients, while a mutation in any of the LRP1B, KRAS, PTPRD, GLI3 and KEAP1 genes is significantly correlated to smoking patients. Similarly, it has been discovered by the present inventors through Fisher's exact tests that a mutation in the EGFR gene is significantly correlated to female patients, while a mutation in any of the LRP1B, APC, KRAS, PTPRD, KEAP1, ITIH5, FLT1 and STK11 genes is significantly correlated to male patients. Likewise, it is shown by the present inventors through Fisher's exact tests that a mutation in the TP53, GLI3 and ITIH5 genes is significantly correlated to patients aged over 60 years, referring to Figure 3.

4.4. Biomarker for predicting prognosis of lung adenocarcinoma in clinical practice

By comparison of lung adenocarcinoma sample data from a cohort population of 335 Chinese patients diagnosed with lung adenocarcinoma, the present inventors found through Kaplan-Meier survival estimation that mutation in a gene selected from any one of the TP53, LRP1B, STK11, KEAP1, BRAF, MET and MRC2 genes indicates significantly shortened survival over a wild-type patient. In the present disclosure, it is proposed that any one of the above seven genes can be used as a biomarker for predicting survival in clinical practice (referring to Figure 4).

**Claims**

1. A method for determining *in vitro* whether a subject suffers from lung adenocarcinoma, comprising:

   detecting whether the MRC2 gene of lung cells or tissue of the subject contains a mutation; and determining that the subject suffers from lung adenocarcinoma when the MRC2 gene contains said mutation.

**2.** A device for carrying out a method as claimed in claim 1 comprising:

a MRC2 gene detecting unit, configured to detect whether a MRC2 gene contains a mutation; and

a MRC2 gene determining unit, connected to the MRC2 gene detecting unit, and configured to determine that the subject suffers from lung adenocarcinoma when the MRC2 gene contains said mutation.

**3.** An *in vitro* method for predicting prognosis of a patient who has undergone treatment for lung adenocarcinoma, comprising:

detecting whether a mutation is present in the MRC2 gene of lung cells or tissues of the patient; and

determining that the patient is in poor prognosis when said mutation is present in the MRC2 gene.

**4.** A device for predicting prognosis of a patient who has undergone treatment for lung adenocarcinoma as claimed in claim 3, comprising:

a MRC2 gene detecting unit, configured to detect a mutation in the MRC2 gene of lung cells or tissues of the patient; and

a MRC2 gene determining unit, connected to the MRC2 gene detecting unit and configured to determine that the patient is in poor prognosis when said mutation is present in the MRC2 gene.

**Patentansprüche**

**1.** Verfahren zur *in vitro*-Bestimmung, ob ein Subjekt an einem Lungenadenokarzinom leitet, wobei das Verfahren folgendes umfasst:

Nachweisen, ob das MRC2-Gen von Lungenzellen oder Lungengewebe des Subjekts eine Mutation enthält; und

Bestimmen, dass das Subjekt an einem Lungenadenokarzinom leitet, wenn das MRC2-Gen die Mutation enthält.

**2.** Vorrichtung zur Ausführung eines Verfahrens nach Anspruch 1, wobei die Vorrichtung folgendes umfasst:

eine MRC2-Gennachweiseinheit, die so gestaltet ist, dass sie nachweist, ob ein MRC2-Gen eine Mutation enthält; und

eine MRC2-Genbestimmungseinheit, die mit der MRC2-Gennachweiseinheit verbunden ist, und die so gestaltet ist, dass sie bestimmt, dass das Subjekt an einem Lungenadenokarzinom leitet, wenn das MRC2-Gen die Mutation enthält.

**3.** *In* vitro-Verfahren zur Abschätzung einer Prognose eines Patienten, der in Bezug auf ein Lungenadenokarzinom einer Behandlung unterzogen worden ist, wobei das Verfahren folgendes umfasst:

Nachweisen, ob eine Mutation in dem MRC2-Gen von Lungenzellen oder Lungengewebe des Patienten vorhanden ist; und

Bestimmen, dass der Patient eine schlechte Prognose aufweist, wenn die Mutation in dem MRC2-Gen enthalten ist.

**4.** Vorrichtung zur Abschätzung einer Prognose eines Patienten, der in Bezug auf ein Lungenadenokarzinom einer Behandlung nach Anspruch 3 unterzogen worden ist, wobei die Vorrichtung folgendes umfasst:

eine MRC2-Gennachweiseinheit, die so gestaltet ist, dass sie eine Mutation in dem MRC2-Gen von Lungenzellen oder Lungengewebe des Patienten nachweist; und

eine MRC2-Genbestimmungseinheit, die mit der MRC2-Gennachweiseinheit verbunden ist, und die so gestaltet ist, dass sie bestimmt, dass der Patient eine schlechte Prognose aufweist, wenn die Mutation in dem MRC2-Gen enthalten ist.

**Revendications**

**1.** Procédé pour déterminer *in vitro* si un sujet est atteint d'un adénocarcinome pulmonaire, comprenant :

la détection de si le gène MRC2 de cellules ou tissus pulmonaires du sujet contient une mutation ; et

la détermination que le sujet est atteint d'un adénocarcinome pulmonaire lorsque le gène MRC2 contient ladite mutation.

**2.** Dispositif pour réaliser un procédé selon la revendication 1 comprenant :

une unité de détection de gène MRC2, configurée pour détecter si un gène MRC2 contient une mutation ; et

une unité de détermination de gène MRC2, connectée à l'unité de détection de gène MRC2, et configurée pour déterminer que le sujet est atteint d'un adénocarcinome pulmonaire lorsque le gène MRC2 contient ladite mutation.

3.  Procédé *in vitro* pour prédire un pronostic d'un patient qui a subi un traitement pour un adénocarcinome pulmonaire, comprenant :

    la détection de si une mutation est présente dans le gène MRC2 de cellules ou tissus pulmonaires du patient ; et
    la détermination que le sujet présente un pronostic défavorable lorsque ladite mutation est présente dans le gène MRC2.

4.  Dispositif pour prédire un pronostic d'un patient qui a subi un traitement pour un adénocarcinome pulmonaire selon la revendication 3, comprenant :

    une unité de détection de gène MRC2, configurée pour détecter une mutation dans le gène MRC2 de cellules ou tissus pulmonaires du patient ; et
    une unité de détermination de gène MRC2, connectée à l'unité de détection de gène MRC2, et configurée pour déterminer que le patient présente un pronostic défavorable lorsque ladite mutation est présente dans le gène MRC2.

Figure 1

Figure 2A

Figure 2B

Figure 2C

Figure 2D

Figure 2E

Figure 3

Figure 4

Figure 5